(19) Europäisches Patentamt
European Patent Office
Office européen des brevets

(11) Veröffentlichungsnummer : **0 159 488**
**B1**

(12) **EUROPÄISCHE PATENTSCHRIFT**

(45) Veröffentlichungstag der Patentschrift : **27.05.87**

(51) Int. Cl.⁴ : **C 07 C 19/02**, C 07 C 17/04

(21) Anmeldenummer : **85102070.1**

(22) Anmeldetag : **26.02.85**

(54) **Verfahren zur Herstellung von 1,2,3-Trichlor-2-methyl-propan.**

(30) Priorität : **25.04.84 DE 3415336**

(43) Veröffentlichungstag der Anmeldung : **30.10.85 Patentblatt 85/44**

(45) Bekanntmachung des Hinweises auf die Patenterteilung : **27.05.87 Patentblatt 87/22**

(84) Benannte Vertragsstaaten : **AT BE CH DE FR GB IT LI NL**

(56) Entgegenhaltungen :
**JOURNAL OF THE AMERICAN CHEMICAL SOCIETY, Band 68, Mai 1946, Seiten 785-789, Easton, GB; A. MOORADIAN et al.: "4-chloro-3-methyl-3-butenonitrile and related compounds"**

(73) Patentinhaber : **HÜLS AKTIENGESELLSCHAFT - RSP Patente / PB 15 - Postfach 13 20 D-4370 Marl 1 (DE)**

(72) Erfinder : **Müller, Dieter Jürgen, Dr. Stargarder Strasse 30 D-4370 Marl (DE)**

**EP 0 159 488 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 1,2,3-Trichlor-2-methyl-propan durch Umsetzung von 3-Chlor-2-methylpropen mit Sulfurylchlorid in Gegenwart von Aldehyden und/oder unter der Einwirkung von Licht.

Die Umsetzung von 3-Chlor-2-methylpropen mit Sulfurylchlorid ergibt Additionsreaktion 1,2,3-Trichlor-2-methyl-propan mit einem Siedebereich von 159 bis 165 °C in einer Ausbeute von 83 % (J. Am. Chem. Soc. 68 (1946) 787).

Dabei wird 3-Chlor-2-methyl-propen in einem Reaktor vorgelegt und die Umsetzung durch allmähliche Zugabe einer insgesamt stöchiometrischen Menge an $SO_2Cl_2$ in der Wärme durchgeführt.

Diese Reaktion ist für ein technisches Verfahren unbefriedigend. Einerseits ist der Start der Reaktion nach Zugabe einer geringen Menge an $SO_2Cl_2$ verzögert und muß durch vorsichtiges Erwärmen in Gang gebracht werden, andererseits besteht die Gefahr, daß die exotherme Reaktion nach dem Start in unkontrollierbarer Weise abläuft.

Darüber hinaus ist die Ausbeute und Selektivität wenig zufriedenstellend.

Die Addition von Chlor an ungesättigte olefinische Verbindungen mittels Sulfurylchlorid kann zwar durch Zusatz von Peroxid oder Radikalbildnern beschleunigt werden (J. Am. Chem. Soc. 61 (1939) 3 432). Von dieser Methode macht man jedoch nur in Sonderfällen Gebrauch, da in der Regel die gleichen Additionsprodukte auch durch Einwirkung von elementarem Chlor leicht zugänglich sind. Die Ausbeuten liegen allerdings nur zwischen 80 und 90 %.

Andere bekannte Reaktionen zur Herstellung von 1,2,3-Trichlor-2-methyl-propan wie die direkte Chlorierung von Isobutan bzw. von Isobuten oder die Weiterchlorierung von geeigneten $C_4$-Chlorkohlenwasserstoffen wie z. B. 3-Chlor-2-methylpropen, 1,3-Dichlorisobuten oder 2-Chlor-2-methylpropen verlaufen noch weniger selektiv.

Der Erfindung liegt die Aufgabe zugrunde, ein Verfahren bereitzustellen, das es gestattet, 1,2,3-Trichlor-2-methyl-propan mit hoher Selektivität in technisch einfacher Weise herzustellen.

Diese Aufgabe wird erfindungsgemäß dadurch gelöst, daß man 3-Chlor-2-methyl-propen mit Sulfurylchlorid in Gegenwart von Aldehyden und/oder unter der Einwirkung von Licht, insbesondere von UV-Licht gemäß folgender Gleichung, bevorzugt im Temperaturbereich zwischen 30 und 65 °C, insbesondere in der Flüssigphase, umsetzt.

$$\underset{\displaystyle Cl}{CH_2} - \overset{\displaystyle CH_3}{C} = CH_2 + SO_2Cl_2 \xrightarrow{RCHO/h.v} \underset{\displaystyle Cl}{CH_2} - \underset{\displaystyle Cl}{\overset{\displaystyle CH_3}{C}} - \underset{\displaystyle Cl}{CH_2}$$

Die Selektivitäten des erfindungsgemäßen Herstellverfahrens liegen mit 91 bis 98 % unerwartet hoch.

Chlorierungsreaktionen mittels Sulfurylchlorid unter der Einwirkung von Licht liefern im allgemeinen neben Chlorsubstitutionsprodukten auch Sulfonsäurechloride, weshalb die durch Radikalbildner katalysierten Chlorierungsreaktionen im allgemeinen unter Ausschluß von Licht durchgeführt werden (J. Am. Chem. Soc. 61 (1939) 2 142).

So sind insbesondere auch selektive Additionsreaktionen mit olefinischen Verbindungen mittels Sulfurylchlorid unter der Einwirkung von Licht nicht bekannt geworden.

Überraschenderweise ist nun gefunden worden, daß 3-Chlor-2-methyl-propen sowohl in Gegenwart von Aldehyden als auch unter der Einwirkung von Licht oder unter der kombinierten Einwirkung von Aldehyden und Licht mittels Sulfurylchlorid mit hohen Selektivitäten von über 90 % in einer Additionsreaktion zu 1,2,3-Trichlor-2-methylpropan umgesetzt werden kann.

Es hat sich herausgestellt, daß Aldehyde bzw. UV-Licht den Reaktionsablauf im gleichen Sinne in Richtung Addition lenken, daß die Selektivitäten und die Reaktionsgeschwindigkeiten jedoch von einander abweichen. Unter der Einwirkung von UV-Licht läuft die Reaktion zwar schneller ab, liefert allerdings eine etwas geringere Selektivität im Vergleich zur Einwirkung von Aldehyden. Bei kombinierter Einwirkung von Aldehyden und UV-Licht resultiert ein Mischeffekt.

Das Sulfurylchlorid kann man, bezogen auf 3-Chlor-2-methyl-propen, in stöchiometrischer Menge einsetzen. Bevorzugt setzt man es im stöchiometrischen Unterschuß ein.

Als Aldehyde eignen sich insbesondere $C_4$-Aldehyde. Der Einsatz anderer Aldehyde ist ebenfalls möglich. Eine vor und/oder während der Reaktion erfolgende Einwirkung von Luftsauerstoff und/oder Wasser auf 3-Chlor-2-methyl-propen stört nicht, da sich dabei im wesentlichen nicht störende Aldehyde bilden.

Im allgemeinen wirken bereits 10 bis 100 ppm der Aldehyde katalytisch. Vorzugsweise werden etwa 100 bis 1 000 ppm zugegeben, obwohl auch höhere Gehalte bis zu 10 000 ppm und mehr möglich sind.

Als Licht eignet sich insbesondere UV-Licht einer Wellenlänge von 200 bis 400 nm.

Das Verfahren eignet sich sowohl für einen diskontinuierlichen Prozeß, beispielsweise in einem Rührreaktor als auch für einen kontinuierlichen Prozeß, beispielsweise in einem Rohrreaktor oder eine Kaskade.

Im Falle der diskontinuierlichen Reaktionsführung wird 3-Chlor-2-methyl-propen vorzugsweise im stöchiometrischen Überschuß in einem heizbaren, kühlbaren Rührreaktor vorgelegt, der mit Belichtungseinrichtung, Rückflußkühler und Einleitungsrohr für Sulfurylchlorid ausgestattet ist. Die Zudosierung des Sulfurylchlorids erfolgt sukzessive in der Weise, daß die bei der exothermen Reaktion entstehende Wärme kontrolliert über die Rückflußkühlung abgeführt werden kann und die Reaktionstemperatur im Bereich von etwa 30 bis etwa 65 °C gehalten wird.

Bei guter Wärmeabfuhr kann die Reaktionszeit auf 1 h oder sogar weniger beschränkt werden.

Die nachfolgenden Beispiele dienen der Verdeutlichung des erfindungsgemäßen Verfahrens.

## Beispiel 1

In einer Rührapparatur mit Rückflußkühler und Tropftrichter, in die eine Hg-Hochdrucktauchlampe (15 W) eingetaucht wird, werden 90,6 g stabilisatorfreies 3-Chlor-2-methyl-propen (Stabilisatorgehalt unter 1 ppm) vorgelegt, auf 45 °C erwärmt und innerhalb von 30 min 108 g $SO_2Cl_2$ unter Thermostatisierung des Rührkolbens zudosiert.

Das bei der Reaktion unter der Einwirkung von UV-Licht freiwerdende gasförmige $SO_2$ und ggf. HCl wird über den Kühler abgezogen. Die Reaktion wird abgebrochen, wenn die $SO_2$-Entwicklung aufhört. Danach wird das Rohprodukt mit Wasser gewaschen, die organische Phase über $K_2CO_3$ getrocknet und zur Ermittlung der Produktzusammensetzung einer gaschromatografischen Analyse unterzogen. Das Ergebnis zeigt Tabelle 1, Zeile 1.

## Beispiel 2

Es wird wie in Beispiel 1 verfahren mit dem Unterschied, daß dem stabilisatorfreien 3-Chlor-2-methyl-propen.

a) 1 000 ppm Isobutyraldehyd bzw.

b) 1 000 ppm Acrolein zugesetzt werden

und die Reaktion unter sonst gleichen Bedingungen ohne UV-Licht durchgeführt wird. Das Ergebnis zeigt Tabelle 1, Zeilen 2a und 2b.

## Beispiel 3

Es wird wie im Beispiel 1 verfahren mit dem Unterschied, daß dem stabilisatorfreien 3-Chlor-2-methyl-propen 1 000 ppm Isobutyraldehyd zugesetzt werden und die Reaktion unter sonst gleichen Bedingungen mit UV-Licht durchgeführt wird.

Das Ergebnis zeigt Tabelle 1, Zeile 3.

## Vergleichsbeispiel 4

Es wird wie im Beispiel 1 verfahren, allerdings wird 3-Chlor-2-methyl-propen ohne jeden Zusatz unter Lichtausschluß mit $SO_2Cl_2$ umgesetzt.

Das Ergebnis zeigt Tabelle 1, Zeile 4.

(Siehe Tabelle 1 Seite 4 f.)

Tabelle 1 : Umsetzung von stabilisatorfreiem 3-Chlor-2-methyl-propen mit Sulfurylchlorid unter Einwirkung von Aldehyden und/oder UV-Licht

| Zeile | Reaktion unter Einwirkung von | Reaktionstemperatur °C | Reaktionszeit incl. Zudosierzeit für $SO_2Cl_2$ Gesamtzeit min | Zudosierzeit min | Rohproduktmenge g | Reinproduktzusammensetzung % +) 1,3-Dichlor-2-methyl-propen | 3-Chlor-2-chlormethyl-propen | 1,2,3-Trichlor-2-methyl-propan |
|---|---|---|---|---|---|---|---|---|
| 1 | UV-Licht | 45 bis 50 | 100 | 30 | 146 | 2,5 | 3,5 | 92,0 |
| 2a | Isobutanal | 45 bis 50 | 180 | 50 | 144 | 1,0 | 0,5 | 97,0 |
| 2b | Acrolein | 45 bis 50 | 180 | 50 | 145 | 1,0 | 1,0 | 96,0 |
| 3 | UV-Licht/ Isobutanal | 45 bis 50 | 130 | 30 | 146 | 1,5 | 1,5 | 95,0 |
| 4 | Vergleichsversuch | 45 bis 50 | 130 | 30 | 133 | 8,5 | 8,5 | 82,0 |

+) Rest auf 100 % nicht näher identifiziert

**Patentansprüche**

1. Verfahren zur Herstellung von 1,2,3-Trichlor-2-methyl-propan durch Umsetzung von 3-Chlor-2-methyl-propen mit Sulfurylchlorid, dadurch gekennzeichnet, daß man die Umsetzung unter der Einwirkung von Licht durchführt.

2. Verfahren zur Herstellung von 1,2,3-Trichlor-2-methyl-propan durch Umsetzung von 3-Chlor-2-methyl-propen mit Sulfurylchlorid, dadurch gekennzeichnet, daß man die Umsetzung in Gegenwart von Aldehyden als Katalysatoren durchführt.

3. Verfahren nach Anspruch 1, dadurch gekennzeichnet, daß man die Umsetzung unter der Einwirkung von UV-Licht durchführt.

4. Verfahren nach Anspruch 1 bis 3, dadurch gekennzeichnet, daß man die Umsetzung unter gleichzeitiger Einwirkung von UV-Licht und Aldehyden durchführt.

5. Verfahren nach Anspruch 1 bis 4, dadurch gekennzeichnet, daß man die Reaktion in Flüssigphase durchführt.

6. Verfahren nach Anspruch 1 bis 5, dadurch gekennzeichnet, daß man die Reaktion bei 30 bis 65 °C unter Normaldruck durchführt.

7. Verfahren nach Anspruch 1 bis 6, dadurch gekennzeichnet, daß man Sulfurylchlorid, bezogen auf 3-Chlor-2-methyl-propen, im stöchiometrischen Unterschuß einsetzt.

8. Verfahren nach Anspruch 2 und 4 bis 7, dadurch gekennzeichnet, daß man als Katalysatoren gesättigte oder ungesättigte Aldehyde im Konzentrationsbereich von 10 bis 10 000 ppm verwendet.

**Claims**

1. A process for the production of 1,2,3-trichloro-2-methylpropane by reaction of 3-chloro-2-methylpropene with sulphuryl chloride, characterized in that the reaction is carried out under the influence of light.

2. A process for the production of 1,2,3-trichloro-2-methylpropane by reaction of 3-chloro-2-methylpropene with sulphuryl chloride, characterised in that the reaction is carried out in the presence of an aldehyde as catalyst.

3. A process according to claim 1, characterised in that the reaction is carried out under the influence of UV light.

4. A process according to any of claims 1 to 3, characterised in that the reaction is carried out under the simultaneous influence of UV light and an aldehyde.

5. A process according to any of claims 1 to 4, characterised in that the reaction is carried out in the liquid phase.

6. A process according to any of claims 1 to 5, characterised in that the reaction is carried out at 30 to 65° under standard pressure.

7. A process according to any of claims 1 to 6, characterised in that sulphuryl chloride is used in less than the stoichiometric amount relative to 3-chloro-2-methylpropene.

8. A process according to any of claims 2 and 4 to 7, characterised in that a saturated or unsaturated aldehyde is used as catalyst in an amount in the range from 10 to 10,000 ppm.

**Revendications**

1. Procédé de préparation de 1,2,3-trichloro-2-méthyl-propane par réaction de 3-chloro-2-méthyl-propène sur du chlorure de sulfuryle, caractérisé par le fait que l'on effectue la réaction sous l'action de la lumière.

2. Procédé de préparation de 1,2,3-trichloro-2-méthyl-propane par réaction de 3-chloro-2-méthyl-propène sur du chlorure de sulfuryle, caractérisé par le fait que l'on effectue la réaction en présence d'aldéhydes en tant que catalyseurs.

3. Procédé selon la revendication 1, caractérisé par le fait que l'on effectue la réaction sous l'action de la lumière ultra-violette.

4. Procédé selon les revendications 1 à 3, caractérisé par le fait que l'on effectue la réaction sous l'action simultanée de la lumière ultra-violette et d'aldéhydes.

5. Procédé selon les revendications 1 à 4, caractérisé par le fait que l'on effectue la réaction en phase liquide.

6. Procédé selon les revendications 1 à 5, caractérisé par le fait que l'on effectue la réaction sous la pression normale, à une température de 30 à 65 °C.

7. Procédé selon les revendications 1 à 6, caractérisé par le fait que, relativement au 3-chloro-2-méthyl-propène, on utilise le chlorure de sulfuryle dans une quantité inférieure à la quantité stœchiométrique.

8. Procédé selon les revendications 2 et 4 à 7, caractérisé par le fait que l'on utilise, comme catalyseurs, des aldéhydes saturés ou non saturés, dans un domaine de concentration de 10 à 10 000 ppm.